(19) **Europäisches Patentamt**
**European Patent Office**
**Office européen des brevets**

(11) **EP 4 670 949 A1**

(12) **EUROPEAN PATENT APPLICATION**
published in accordance with Art. 153(4) EPC

(43) Date of publication:
**31.12.2025 Bulletin 2026/01**

(51) International Patent Classification (IPC):
**B29C 48/00** *(2019.01)*  **B29C 48/92** *(2019.01)*
**G01N 33/44** *(2006.01)*  **B29B 17/00** *(2006.01)*
**B29C 48/27** *(2019.01)*  **B29C 48/275** *(2019.01)*
**B29B 7/38** *(2006.01)*  **B29B 7/72** *(2006.01)*
**B29K 23/00** *(2006.01)*  **B29K 67/00** *(2006.01)*
**B29K 7/00** *(2006.01)*  **B29K 105/00** *(2006.01)*
**B29K 105/26** *(2006.01)*  **B29L 7/00** *(2006.01)*
**B29C 48/08** *(2019.01)*

(21) Application number: 24713682.3

(22) Date of filing: 21.02.2024

(52) Cooperative Patent Classification (CPC):
**B29C 48/022; B29C 48/92; G01N 33/442;**
B29B 7/38; B29B 17/0042; B29C 48/08;
B29C 48/277; B29C 2948/92228;
B29C 2948/92333; B29C 2948/92723;
B29C 2948/92828; B29K 2023/06; B29K 2023/0633;
B29K 2023/065; B29K 2023/083;       (Cont.)

(86) International application number:
**PCT/ES2024/070103**

(87) International publication number:
**WO 2024/175822 (29.08.2024 Gazette 2024/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.02.2023 ES 202330145**

(71) Applicant: **CADEL RECYCLING LAB, S.L.**
**46893 Alfarrasi (Valencia) (ES)**

(72) Inventors:
• **GARCIA VIDAL, Rafael**
**46893 Alfarrasi Valencia (ES)**

• **TAMARIT GRAMAGE, Sergio**
**46893 Alfarrasi Valencia (ES)**
• **COLOMO GAVILAN, Lorenzo**
**46893 Alfarrasi Valencia (ES)**
• **GOMEZ MARTINEZ, Melania**
**46893 Alfarrasi Valencia (ES)**
• **OVCHINNIKOV, Konstantin**
**46893 Alfarrasi Valencia (ES)**
• **AMAT BERNABEU, Adrián**
**46893 Alfarrasi Valencia (ES)**
• **PINEDA VAZQUEZ, Adriana**
**46893 Alfarrasi Valencia (ES)**

(74) Representative: **Temiño Ceniceros, Ignacio**
**Abril Patentes y Marcas, S.L.**
**Calle Zurbano, 76 - 7°**
**28010 Madrid (ES)**

(54) **METHOD FOR MANUFACTURING A THERMOPLASTIC MATERIAL WITH A DETERMINED LEVEL OF CONTAMINATION**

(57)     The method comprises determining the level of contamination, in a particular embodiment, by sequentially executing the following steps: (a) characterizing the recycled input material; (b) calculating the level of contamination (LC) from the characterization data of the material and/or images; and (c) calculating the percentage of recycled material to be introduced into the batching system of a plastic material extruder; and wherein image capturing may be performed, for the manufacture of a recycled plastic film with a known LC.

(52) Cooperative Patent Classification (CPC): (Cont.)
B29K 2067/003; B29K 2067/046; B29K 2105/256;
B29K 2105/26; B29L 2007/00; Y02W 30/62

## Description

### Technical field

The present invention relates to a method, a system and a computer program product for determining the level of contamination of a recycled plastic. Therefore, the invention falls within the technical field of plastic recycling and, in particular, a polyester plastic and polyolefin derivatives.

The level of contamination (LC) in materials is defined by multiple parameters that include chemical composition, assessed through the identification of organic substances, including those listed under regulations such as those established by the European Chemicals Agency (ECHA) and substances of very high concern (SVHC); physical aspects such as the detection of gels, luminosity of the material, the coloring thereof, among others; mechanical properties that include impact, tear, and traction tests, among other methods; as well as rheological properties, including flow rate. This multidimensional approach allows for a comprehensive and accurate assessment of the level of contamination, which is essential for making decisions in various fields, mainly in the plastic manufacturing industry.

### Prior art

Reducing the use of virgin plastic has been encouraged over the last 10 years. This fact is even more notable following the announcement of the increase in the rate on virgin plastic in Spain, as well as the limitations established by Europe regarding the manufacture of single-use plastic waste. Social awareness of plastic recycling has experienced a considerable increase, with the purpose of promoting a sustainable circular economy, preserving the environment and mitigating global warming. In this sense, for some years now, sustainable applications are being developed that will generate recycled plastic products with high added value.

Within the plastics industry, sectors such as food and hygiene use large quantities of different types of plastic to package their products, including PET and polyolefins, such as LDPE, HDPE, among others. To that end, recycled material is incorporated under thorough quality controls to ensure the safety of the material within the relevant application, as well as to ensure compliance with the regulations of each sector.

With regards to flexible packaging, the plastic film manufacturing process is based on a mechanical industrial process, called extrusion, which is configured to mold the plastic material through a common flow of pressure and thrust. The plastic polymer in the form of pellets is melted, acquiring a viscoelastic state, and passed through a mold where it acquires the desired shape.

To maximize the use of recycled content and avoid problems during the extrusion process, such as the breakage of plastic film or the clogging of filters, certain quality criteria are established, which depend on the user, and are mainly based on the physicochemical properties of recycled plastic, such as the number of gels or the luminosity parameter, among others, added to the manufacturer's own experience. In this sense, the existence of technologies implemented within the plastic film manufacturing process, wherein the classification of recycled material is normalized and the batching thereof is automated, depending on the type of batch and its quality in terms of contamination, is unknown.

In the context of the characterization and quality control system in the recycling industry, it is noted that these processes are mostly carried out manually, involving a significant time commitment and increasing the likelihood of human errors. These conditions generate inefficiencies and discrepancies in product inspection.

The lack of capabilities for real-time monitoring and analysis makes it difficult to promptly identify and resolve quality problems, which inevitably affects product quality and customer satisfaction. For example, document EP1055500 discloses a method for manufacturing recycled material from plastic material, but wherein the technical problem of controlling quality to improve the extrusion process is not addressed.

Traditional quality control methods may encounter obstacles in keeping up with the changing demands of industries, resulting in outdated practices and suboptimal results.

The present invention aims to provide a new approach in the plastic products manufacturing process by means of extrusion, characterizing the recycled plastic according to its degree of contamination, providing the necessary information to design and readjust plastic film extrusion recipes that incorporate recycled material content.

Furthermore, the present invention automates quality control tasks, reducing dependence on manual inspection and minimizing the risk of errors, which increases the effectiveness and consistency of the quality assurance process. Given the functions of real-time (online mode) or offline data monitoring and analysis, it provides visibility into quality metrics, enabling proactive identification of defects and taking immediate corrective action to maintain high product standards.

This solution is described in the claims accompanying this specification.

## Description of the invention

An object of the present invention is a method for determining the level of contamination of recycled plastic, such as, for example, with regards to its chemical composition at the level of substances of interest or concern according to the lists provided, among others, by the European regulation on plastic materials in contact with food EU FCM 10/2011 and ECHA, as well as the automation and implementation of a batching system incorporated in a plastic extruder to pre-establish the conditions required to obtain a plastic film with an adequate quality and level of contamination, based on the acquisition of data or images previously correlated with analytical techniques, such as gas chromatography (GC-MS) and/or liquid chromatography (LC-MS), thermogravimetric analyses such as TGA, DSC, among others, as well as spectroscopic techniques such as FTIR, among other polymeric material characterization techniques.

Another object of the invention is to establish a quality control system for recycled material, helping to create the recipes necessary for taking material during the extrusion of a new plastic, in order to predict the level of contamination that is obtained when different combinations of virgin plastic and recycled plastic are used.

Another object of the invention is to determine the level of contamination of recycled plastic, at least one computer program composed of a plurality of instructions stored in a memory and that, when executed by a processor, cause the system to establish the level of contamination of each available batch and calculate the amount of plastic that must be batched in the extruder (i.e., the ratio between virgin and recycled plastic material with different degrees of contamination) so that the resulting plastic film reaches the initially preestablished quality conditions, maximizing the use of recycled material. The computer program can be fed both by data generated through physical, chemical and mechanical characterization of the material and by images.

These objects are achieved with the method of claim 1. Other aspects of the invention, as well as different practical embodiments are described in the claims that are directly or indirectly dependent on claim 1.

More specifically, the invention is based on a thermoplastic material in the form of pellets (granules), flakes, sheet (film) or preform (in case of PET), where the input material is, for example, polyethylene (PE) and derivatives thereof such as LDPE, PP or HDPE, PET, PLA, EVA, or others, or a combination thereof. The level of contamination is determined by sequentially executing the steps of: (a) characterizing the recycled input material; (b) calculating the level of contamination (LC) from the characterization data of the material and/or generated images; and (c) calculating the percentage of recycled material to be introduced into the batching system of a plastic material extruder for the manufacture of a recycled plastic film with a known LC.

The characterization of the material comprises at least one of the following analyses: (a) analytical (identification of pollutants using liquid chromatography mass spectrometry (LC-MS) or gas chromatography mass spectrometry (GC-MS)); (b) physical (measurement of color, turbidity and gel count); (c) mechanical (tension, friction); and (d) toxicity studies (risk assessment for food contact applications); and it can (e) capture images.

The first step is based on defining the most relevant parameters and the parameters that have the most influence on the labeling of the plastic material sample. Thanks to this characterization step, it is possible to develop a statistical method that groups recycled plastic material according to its quality based on its level of contamination.

Once the plastic material, preferably in pellet format, is received, a sampling and characterization process is performed. Based on the type of analysis to be performed, the material will be used in the form of pellets (analytical, physical, toxicity analysis) or as a film (mechanical tests). The following parameters are determined with the homogenized plastic material.

In any case, it must be taken into account that the plastic material can be presented in the form of a sheet, granules, flakes or preform (in the case of PET plastic material). Depending on the type of analysis to be performed, the homogenized material will be used in the form of granules for analytical, physical and toxicity analyses, or in the form of a film for mechanical tests. In the case of image capturing, the system is compatible with any format, although the sheet (film) format is preferred.

The different steps are described below according to the type of material and the type of data available.

- In the *analytical analysis,* the homogenized material is subjected to an extraction process using an organic solvent (in a particular embodiment, MeOH, EtOH, DCM, THF, among others) or aqueous solvents of different groups of substances such as Bisphenol A (BPA), phthalates, alkylphenols (AP), polycyclic aromatic hydrocarbons (PAHs), derivatives of aromatic amines, alkyl amines, among others. Next, the study on identifying volatile, semi-volatile and non-volatile substances is carried out through the use of individual or joint techniques, GC-MS, LC-MS and, for example, the identification of elements, such as heavy metals through the use of ICP-MS.
- In the *toxicity analysis,* the risk assessment is carried out, considering whether the substance is CMR (carcinogenic, mutagenic or reprotoxic), if it is classified as a substance of very high concern (SVHC) by ECHA (European Chemicals Agency), as well as the maximum permitted concentration level and the specific migration limit (SML) in accordance with the regulations of each jurisdiction, such as, for example, the EFSA in Europe or the FDA in the United States of America, for plastic products in contact with food.

- The *physical analysis* comprises the characterization of the physical properties of the material through, at least, the measurement of color, opacity and gel count.
- The *mechanical analysis* includes, at least, a friction and traction test of the recycled plastic material film.
- The *obtaining of images* of a plastic sample is performed by using tools such as microscopes or multispectral cameras, among others. These images are subjected to processing to normalize the data that will be sent to the system. The initial image may consist of the very chromatogram obtained during the analytical contamination test, with the purpose of establishing a correlation between the image and its level of contamination.

With all the information obtained, a data table is created and a level of contamination (LC) is statistically classified and assigned for each batch. This level of contamination is expressed as the percentage of recycled material to be incorporated into the extrusion process by comparing the levels of contamination of the different available batches and readjusting the batching according to the LC value established for the final product.

Lastly, it should be noted that since that the combination of materials for the same level of contamination can be infinite, minimizing the contribution of virgin material or maximizing the incorporation of recycled material is established as a criterion.

Throughout the description and the claims, the word "comprises" and its variants are not intended to exclude other technical features, components or steps. For those skilled in the art, other objects, advantages and features of the invention may be inferred from both the invention and the embodiment of the invention. The following examples and drawings are provided by way of illustration and do not seek to limit the present invention. Furthermore, the invention covers all possible combinations of particular and preferred embodiments indicated herein.

**Brief description of the drawings**

Next, a series of drawings that help to make the invention more readily understandable and that are expressly related to an embodiment of said invention, which is illustrated as a non-limiting example thereof, is briefly described below.

Figure 1 shows a diagram of an exemplary practical embodiment of the method of the invention, along with the elements that make up the system.

Figure 2 shows the algorithm for determining the percentage of optimal recycled plastic material using an unsupervised model, according to the present invention. Figure 3, with its images Figure 3.1., Figure 3.2. and Figure 3.3., shows a flowchart with the decision tree according to toxicological criteria.

Figure 4 schematically shows that the image obtained directly from the chromatogram can serve as a basis to feed the system.

Figure 5 schematically shows the steps for classifying recycled material by image capturing.

Figure 6 shows confusion matrices obtained through SVM approximation (figure 6, left) and Random Forest (figure 6, right).

**Detailed explanation of an embodiment of the invention**

The method of this exemplary embodiment is based on a thermoplastic material, in granular format, flakes, sheet or preform (in case of PET), mainly in the form of pellets. The input material can be polyethylene (PE) and derivatives of same such as LDPE, PP or HDPE, PET, PLA, EVA, or a combination thereof.

As stated previously, the method of the invention consists of the following steps:

- Characterizing the recycled input material.
- Training the mathematical algorithm.
- Exporting data or images and calculating the level of contamination (LC) from the characterization data of the material.
- Calculating the percentage of recycled material by adjusting the extrusion parameters to be used based on the quality of the recycled plastic.

*Characterization of the recycled input material by means of its chemical, physical and mechanical composition*

As a representative, but non-limiting example, in the first step, the size of the sample to be analyzed is calculated so that it is representative of the total batch. To estimate an average, the following equation is used:

$$n = \frac{Z_\alpha \cdot \sigma}{p}$$

where n is the sample size (in sample units), $Z_\alpha$ is the value corresponding to the desired confidence (e.g., $Z_\alpha$ = 1.96 for 95% confidence and $Z_\alpha$ =2.576 for 99% confidence); $\sigma$ is the standard deviation of the population, such that this value may vary depending on the plastic. Standard deviations have been observed in the range of 0.6 $\frac{\mu g}{kg\ plastic}$ to 345.9 $\frac{\mu g}{kg\ plastic}$ ; and p is the measurement accuracy, in other words, of the chromatograph.

Once the required sample quantity has been calculated, the number of units required (pellets) can be calculated by multiplying the amount of sample needed by the weight of each unit. An average value used is 0.02425 g/unit.

$$m\,(g) = 0.02425 \frac{g}{unit} \cdot n$$

The sample size determined in the first step is important to ensure adequate representativeness of the sample and acceptable accuracy in the analysis results.

Once sampling is established, the characterization of the plastic begins. The chemical composition is carried out using chromatographic techniques, where the accuracy and sensitivity of analytical measurements allow the concentration of volatile substances in the sample to be determined accurately and reliably. The compounds included, such as bisphenols, phthalates, PAH (Polycyclic Aromatic Hydrocarbon) and AP (Alkylphenols), among others, are identified and separated based on their physical and chemical properties, such as retention and molecular mass. The result is a graphic representation of the compounds present in plastic, called a mass profile, which makes it possible to identify and quantify in units of:

$$\frac{\mu g\ substance}{kg\ plastic}$$

the presence of these compounds in plastic.

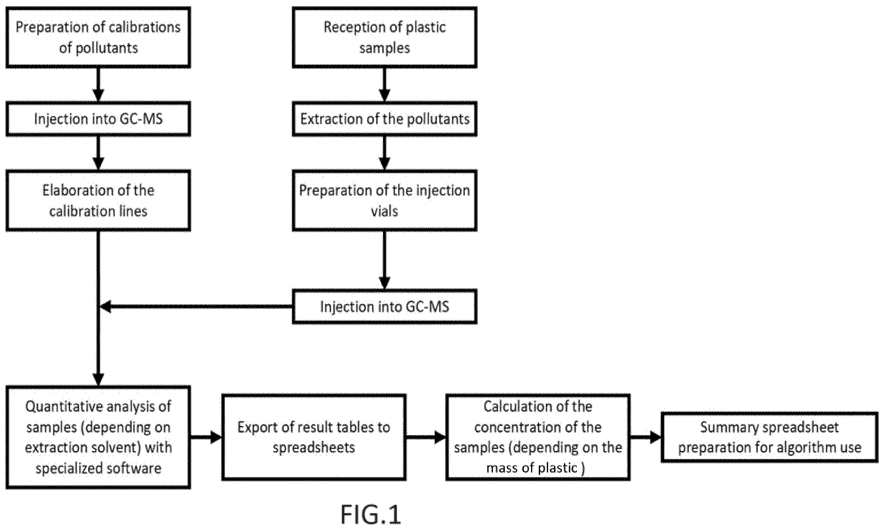

Figure 1 outlines the different steps to follow in the analytical characterization of the material. In this sense, to know the content of the different pollutants present in the plastic samples, extraction preprocessing with an organic solvent is required in which the group of compounds to be analyzed is completely soluble.

In case of groups of compounds with different affinities, different portions of the target sample in the solvents will be extracted as necessary until all the volatile compounds to be determined are extracted. For example, obtaining extracts of non-polar and polar substances with a non-polar solvent and a polar solvent, respectively and independently.

In general, the concentrations of the compounds to be determined are of very low magnitudes. In chromatographic equipment, slight variations in sensitivity may occur between sequences of samples to be determined. This is why an accurate and suitable quantification technique must be selected.

Both for the standards used for the calibration curves and for the samples to be quantified, the internal standard method is applied, which consists of adding a compound of a known and fixed concentration and having a similar nature to the analyte to be determined, but which is not present in the sample nor does it react with same. This allows the signals obtained from the samples to be normalized on a substance which concentration is known to remain unchanged.

Once the vials containing the sample and internal standard have been prepared, they are taken to the corresponding analytical apparatus (GC-MS, LC-MS, or ICP, among others), which is connected to a computer that has a computer program provided by the same distributor of the equipment with which the sequence of vials to be injected and the chromatographic method of the group or groups of compounds to be quantified can be created.

As a representative example, in GC-MS (gas chromatography coupled to mass spectrometry), when the separation and ionization of the compounds of a sample ends, the program returns a folder that includes all the information necessary to plot its chromatogram and obtain the mass spectra. In said chromatogram, the signal obtained with the passage of the different analytes is represented as a function of their retention time (RT) in the gas column. Meanwhile, the mass spectra represent the fragmentation of the different ions and their relative abundance at a retention time or retention time range of the corresponding chromatogram. In order to graphically represent this information, the very GC/LC-MS equipment has specialized programs that load the folders created from each injected vial and perform the conversion automatically, leaving the detection and quantification of the compounds as a task for the user in this part of the process.

Before beginning the quantitative analysis, it is first necessary to create calibration lines for the compounds of interest in which the signals obtained from each sample are to be interpolated. To that end, standards of the compounds to be measured at different known concentrations and in a concentration range in which they are estimated to be present in the samples are prepared. As mentioned above, when working with the internal standard method, the internal standard of the group of substances corresponding to the same final concentration as that added to the samples will also be added to these calibration points.

This graphical representation of the analyte signal (normalized with the internal standard signal) as a function of concentration gives an equation of the line that will be used to calculate sample concentrations. There are specialized computer programs that facilitate the creation of the calibration line and the calculation of the concentration value.

The values of the signals used for the calibrations and quantitative analyses are taken from the area obtained from the integration carried out under the curve of the chromatographic peaks of the corresponding pollutants since, as a variable, this is more accurate than the height of the peaks. Thus, the general calibration equation will have the following form:

$$A_{analyte}\Big/_{A_{PI}} = f(C_{analyte})$$

Where $A_{analyte}$ corresponds to the area under the curve of the analyte; $A_{PI}$ corresponds to the area under the curve of the added internal standard; and $f(C_{analyte})$ corresponds to the concentration of the analyte in the injected vial (in mass of analyte per volume of solution).

Given the behavior of some pollutants, in certain cases, calibrations may need to be adjusted to a quadratic rather than a linear format to obtain a suitable regression. Once the calibration lines have been prepared, it is possible to proceed to the quantification step.

It should be noted that a chromatogram, which represents a data table of substances compared to their concentration, can be used as an image for its inclusion in the digital tool.

*Quantification of the substances identified in the sample*

In the quantification of the identified substances, the peaks observed in their chromatograms (as well as that of the corresponding internal standard) should be integrated and the calibration equation should be used to obtain the concentration values. Since the aim is to obtain the concentration value of the pollutants in the plastic itself and not in the solvent that has been used solely as an injection means, the units reported by the chromatograph must be converted into units applicable to the algorithm. If, for example, during the injections work was carried out in units of micrograms of substance per liter of solution and there is a desire to convert them into milligrams of substance per kilo of plastic, the following conversion must be performed:

$$\frac{\mu g\ substance}{L\ solution} \cdot \frac{1\ mg\ substance}{1000\ \mu g\ substance} \cdot \frac{L\ solution}{Kg\ plastic}$$

All this information is exported in a table that comprises at least the following information:

| Group of Compounds | | Sample | | | Internal Standard | |
|---|---|---|---|---|---|---|
| Name | CAS | RT | Area | Concentration | RT | Area |
| | | | | | | |

Since each group of compounds to be analyzed is quantified in individual batches, the tables exported by groups are first separated. For example, data exported from PAHs is processed separately from data exported from APs. Likewise, the samples are separated by spreadsheets, within which all the exported replicas will be included, as well as the calculations carried out to obtain the concentration values of said sample based on the mass of plastic, taking into account the masses of plastic used for each vial and the volume of solvent used for each extraction:

| Sample A | | | | | | |
|---|---|---|---|---|---|---|
| Compound | Replica | $\dfrac{\mu g\ substance}{L\ solution}$ | Plastic mass (kg) | Solution volume (L) | $\dfrac{mg\ substance}{Kg\ plastic}$ | Mean (mg/Kg plastic) $\pm$ SD |
| | 1 | | | | | |
| | 2 | | | | | |
| | 3 | | | | | |

Said set of samples, once processed, will include only the mean and standard deviation (SD) values, and it will be indicated if the values obtained are below the limits of quantification (LOQ):

| Compound | CAS | Sample A (mg/Kg plastic) | | Sample B (mg/Kg plastic) | | Sample C (mg/Kg plastic) | |
|---|---|---|---|---|---|---|---|
| | | Mean $\pm$ SD | | Mean $\pm$ SD | | Mean $\pm$ SD | |
| | | | | | | | |

These equipment LOQs are determined from the calibrations prepared for each group of substances, where the point of lowest concentration is defined as said limit, below which it is not possible to ensure the concentration values obtained from the quantitative analysis. Once the data of all the groups of compounds of interest have been processed, a single normalized table is established with all the necessary data to be used in the next step:

| | | | Sample A (mg/Kg plastic) | Sample B (mg/Kg plastic) | Sample C (mg/Kg plastic) |
|---|---|---|---|---|---|
| Group of compounds | Compound | CAS | Mean $\pm$ SD | Mean $\pm$ SD | Mean $\pm$ SD |
| Phthalates | | | | | |
| | | | | | |
| | | | | | |
| Group of compounds | Compound | CAS | Mean $\pm$ SD | Mean $\pm$ SD | Mean $\pm$ SD |
| BPA | | | | | |
| AP | | | | | |
| | | | | | |
| | | | | | |
| PAH | | | | | |
| | | | | | |
| | | | | | |

*Data processing*

The software is designed to adapt based on the input data entered, whether they be data and/or images.

In this phase, the acquisition and processing of data is carried out by information collection, analysis and organization. The compounds and chemical substances identified and quantified in the previous step are stored and transferred to a database for subsequent analysis. Subsequently, this data is integrated into the system, where the extrusion process is adjusted to determine the optimal ratio between virgin and recycled material, with the aim of meeting the demanding quality standards established.

The algorithm used to manipulate the mixture and obtain a desired concentration level that works correctly in the extruder and meets regulations is a process of mathematical optimization. The present invention uses two types of models, preferably statistical, that are presented below. In this sense, during the tool training process and with the aim of ensuring optimal operation, the algorithm or a combination thereof that has demonstrated an optimal correlation through mathematical optimization is selected. This allows the parameters of the extrusion process to be adjusted digitally or automatically throughout the entire process.

Next, the two types of statistical models used in the present invention are described.

*Supervised model*

In addition to unsupervised learning algorithms, it is also possible to use supervised learning algorithms to classify the quality of recycled plastic. In this case, prior labels would be provided to a set of training data, and the algorithm would learn to classify new samples based on these labels.

Training would consist of providing the supervised model with a large set of input data obtained from the chromatograph, of product images (for example, for visual recognition of the recycled product or the virgin product) and mechanical and physical aspects. This data includes information on the concentration of the compounds in the recycled plastic and other relevant aspects, such as the luminosity of the pellets, luminosity of the plastic film, increase in pressure in the filters, or any others.

The supervised model will learn to identify standards in the data and associate these standards with the appropriate quality labels. During training, the model is exposed to a wide variety of data examples and labels, and it adjusts until it can correctly classify the data. Once trained, the model can be used to classify the quality of recycled plastic in new samples analyzed by the chromatograph. The QD is the final result of the classification and is used to determine the quality of the recycled plastic sample.

*Supervised model using neural network*

The classification process of plastic quality with a supervised neural network model comprises:

(a) Data collection, of both the composition of the plastic through the chromatograph and the mechanical and physical aspects described (pressure, usage time, melting temperature and advances).
(b) Preprocessing of the data for its normalization and preparation for model training.
(c) The creation of the neural network model with a suitable structure for the problem of classifying plastic quality. The structure of a supervised neural network to classify plastic quality would be based on:

(c.1) an *input layer* that will receive the input data obtained from the chromatograph and mechanical and physical aspects. This data is normalized and presented in the form of vectors;
(c.2) one or more hidden layers that process the input data and learn the relationships between them by using an activation function. The number of hidden layers and the number of nodes in each layer can vary depending on the complexity of the problem; and
(c.3) an output layer that provides the quantum distance (QD) as a result and provides the classification of plastic quality.

(d) Training of the model with the preprocessed data, using optimization and backpropagation techniques to update network weights and improve classification accuracy.
(e) Lastly, the model would be assessed with a set of test data to assess its accuracy and determine its capability to generalize to new cases.

Once trained, the neural network model could be used to classify new cases of plastic and label them with an appropriate quality category, based on the quantum distance (QD) that is calculated from the input data.

*Supervised Bayesian model*

A Bayesian model uses Bayes' Theorem to estimate the probability of an event given a set of data and a hypothesis. In the case of the classification of plastic quality, the Bayesian model would use the data obtained from the chromatograph and other mechanical and physical aspects to estimate the probability that a plastic sample belongs to a certain class or label (for example, A, B, C or D).

The Bayesian model would be trained with a set of pre-labeled data, so that it can learn to associate certain input values with certain labels. Then, when new data is provided, the model will calculate the probability that they belong to each class and assign the label with the highest probability.

The Bayesian model can be an appealing alternative to neural networks in situations where data is limited or when a clearer interpretation of the results is required, since Bayesian models are usually more explainable and based on sound probabilistic theory.

*Decision tree model*

A decision tree model would work to classify plastic quality by collecting the data extracted from the chromatograph and information from mechanical and physical aspects. The model would create a tree with different nodes and branches that represent decisions based on the relevant variables. Each node or branch of the tree represents a decision based on the comparison of a variable with a threshold value.

The quality label is assigned on the final leaf of the tree, which represents the final classification of the plastic. For example, one could start by comparing the quantum distance obtained from the chromatograph with a threshold value. If the quantum distance is greater than the threshold value, the classification is directed along a branch towards a node that represents the comparison of the melting temperature with another threshold value. This process would be repeated with other relevant factors until reaching the final leaf and assigning an appropriate label to the quality of the plastic.

*Unsupervised model*

This process uses a target function, which represents the desired concentration level, and one or more constraints, which represent the limits of the available concentrations. In other words, it is a multi-target problem in which there are more criteria to optimize. In this case, the problem is that of finding an optimal solution for two targets:

   a) Mechanical constraint based on the QD variable.
   b) Compliance with toxicological standards.

These targets may be contradictory, in other words, optimizing one target can negatively affect the other. The problem is addressed through a mathematical optimization process that uses a target function and constraints. The final solution is an optimal mixture that meets quality requirements and both mechanical and toxicological constraints. The target function is optimized to meet the constraints and reach the desired concentration level efficiently. This manipulation through the use of software that received the concentration data of the compounds in the plastic allows obtaining an optimal mixture that meets the desired quality requirements.

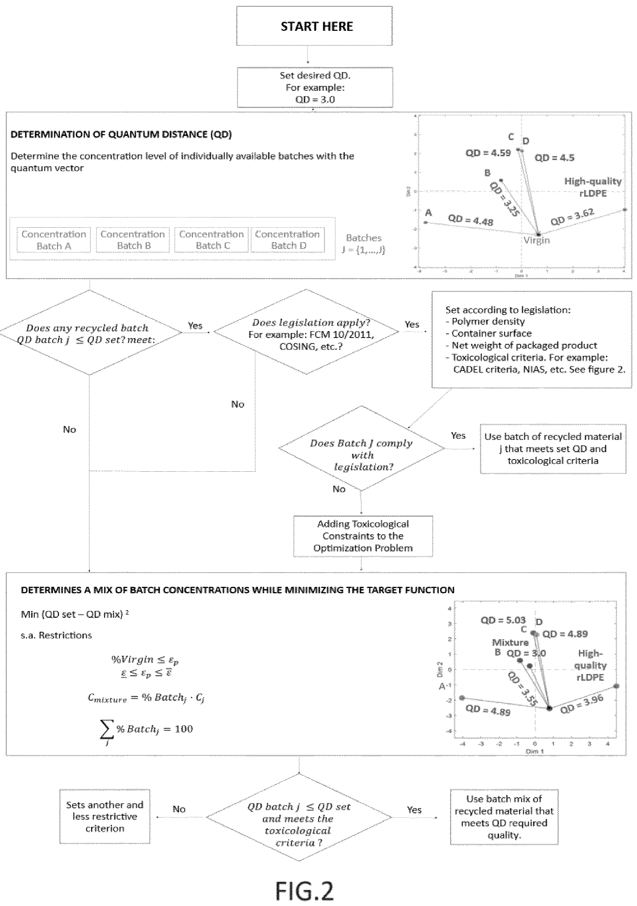

The previously described algorithm is shown in figure 2. Next, the steps that the algorithm takes into account are briefly explained: (a) a desired value for the quantum distance (QD) is set. This value will in turn be associated with a level of contamination (LC) and some mechanical and physical parameters; (b) the algorithm asks whether the relevant legislation applies to the intended application; if the answer is yes, additional parameters are set such as the density of the polymer, the surface of the container and the net weight of the packaged product, and a toxicological criterion is applied. If the answer is no, the quantum distance is determined; (c) the concentration levels of the individual available batches are compared with the quantum vector. If the batch meets the set QD, the final application of the material is defined. If used in contact with food, a toxicological criterion is applied, estimating the suitable mixture of concentrations of the different batches, so that it minimizes the target function.

In cases in which the application is not related to contact with food, the next step would be to directly determine the percentage of recycled material that can be incorporated into the extrusion. It is checked whether the batch mixture meets the required quality (QD) and the toxicological criterion. If it does, the mixture is used. If not, a less restrictive criterion is set and the process begins again from step (b).

*Calculation of quantum distances (QD)*

To determine the concentration level of recycled plastic pellets, a classification algorithm based on unsupervised learning that collects the data extracted from the chromatograph, or images of the samples being analyzed, or a combination thereof, is used. The information is processed to obtain a quantum distance that represents the concentration level of recycled plastic in the analyzed sample. The classification is based on comparing the quantum vector with previously identified standards (virgin and high-quality plastic) and assigning a corresponding concentration level.

The quantum distance is useful because it provides a simplified and summarized representation of the concentration of the different substances present in the plastic. This allows for easier and faster assessment of material quality, especially when there are hundreds of substances present and their exact origin or composition is not known. Furthermore, it can also be used as a monitoring tool to verify the quality consistency of different batches of recycled material.

Next, a possible embodiment of the calculation is developed to determine the quantum distance based on the distance between the virgin plastic sample and the recycled plastic sample using the method described below.

(a) First of all, a data matrix should be prepared that includes the concentration values of bisphenol, PAH and AP, or any others that are considered for each sample (reference plastic, batch A and batch B, batch C, ..., batch J) detected and quantified with GC-MS.

| Batch | BPA $\left(\dfrac{\mu g}{kg \; plastic}\right)$ | PAH 1 $\left(\dfrac{\mu g}{kg \; plastic}\right)$ | PAH 2 $\left(\dfrac{\mu g}{kg \; plastic}\right)$ | AP $\left(\dfrac{\mu g}{kg \; plastic}\right)$ |
|---|---|---|---|---|
| Reference plastic | 1.2 | 3.5 | 2.1 | 1.1 |
| Batch A | 2.5 | 3.2 | 3.3 | 21.2 |
| Batch B | 3.5 | 3.6 | 6.4 | 8.2 |
| Batch C | 23.2 | 32.1 | 2.1 | 2.1 |
| Batch J | 3.5 | 5.2 | 3.6 | 33.3 |

(b) Data normalization: The data must be normalized to ensure that the variables have the same importance and scale in the analysis. For this, the following formula is used:

$$\hat{d} = \frac{\mu - d}{\sigma}$$

Where $\hat{d}$ is normalized data, $\mu$ is the mean concentration of the variable, $\sigma$ is the standard deviation of the variable.

(c) The covariance matrix of the normalized data or the correlation matrix is calculated:

$$A = \frac{1}{n-1} \cdot \hat{d}^T \cdot \hat{d}$$

Where A is the correlation matrix, $\hat{d}$ is the normalized data matrix, and n is the number of samples

(d) The eigenvectors and eigenvalues of the correlation matrix are found. The general formula for calculating the eigenvalues is:

$$|A - \lambda I| = 0$$

Where A is the correlation matrix, $\lambda$ is the eigenvalue, I is the identity matrix. Moreover, the eigenvectors are calculated by solving the following equation:

$$Av = \lambda v$$

Where Av is the normalized data matrix multiplied by the vector v, $\lambda$ is the corresponding eigenvalue and v is the eigenvector.

(e) The eigenvalues are arranged in decreasing order and the eigenvalues corresponding to the largest eigenvalues are chosen.

(f) The arranged eigenvectors are multiplied by the correlation matrix to obtain the quantum vectors S.

$$S = v \cdot \hat{d}$$

(g) Calculation of quantum distances (QD), where once point VI is reached, the quantum distance (QD) can be calculated from the matrix S of quantum vectors. The Euclidean distances between the reference plastic samples and each of the recycled plastic samples are calculated using only the first two dimensions.

$$S = \begin{bmatrix} x_{ref} & y_{ref} & z_{ref} & \cdots \\ x_A & y_A & z_A & \cdots \\ x_B & y_B & z_B & \cdots \\ \vdots & \vdots & \vdots & \ddots \\ x_j & y_j & z_j & \cdots \end{bmatrix}$$

$$QD_j\,(x,y) = \sqrt{(x_{ref} - y_{ref})^2 + \left(x_j - y_j\right)^2}\;\forall\,j$$

Where x and y are the first two components of the quantum vectors.

*Determination of batch mixing percentage*

Preferably, the optimization problem that is solved is the batching percentage of each batch in such a way that the mixing percentages of each batch are adjusted to obtain a specification that is as similar as possible to the specification that is set.

In other words, specified mathematically, a target function that takes into account the calculation of the quantum distance of the mixture mixture QD (as explained above) and the set quantum distance set QD is minimized.

$$\text{Min FO} = (\text{set QD} - \text{mixture QD})^2$$

The concentration of the mixture is the mean concentration of the initial batches.

$$C_{mixture} = \%\,Batch_j \cdot C_j$$

Furthermore, constraints on the maximum or minimum amount of each component that can be used, or on the minimum or maximum ratio between different components, could be included. Other constraints could include conditions of equality or inequality on the optimization variables, limits on the range of the variables, or any other requirement specific to the problem being addressed. These constraints should be included in the formulation of the optimization problem to ensure that the solution meets the desired requirements.

The optimization algorithm used by default is Sequential Quadratic Programming (SQP). This algorithm is a non-linear optimization method that combines solving a quadratic programming problem at each iteration with a linear search to find a local minimum of a non-linear target function.

*Toxicological criterion*

Information about the concentration of volatile substances in the samples is stored in the SQL database. The date of sample collection, the type of recycled material (type of subsequent consumption) and its format (pellet, scales or other) is recorded. The following variables are recorded on each of the substances identified in a sample:

- CAS number.
- Names of the compound.
- Concentration in the plastic (mg/Kg, $\mu$g/Kg, ng/Kg).
- LOQ ($\mu$g/Kg): limit of quantification of the substance.
- LOD ($\mu$g/Kg): limit of detection of the substance.

With this information, the software will calculate migration values (assuming 100% migration from packaging to food) and EDI (Estimated Daily Intake, for products in contact with food) using the formulas:

$$\text{Theoretical migration}_{\text{assuming migration of }100\%}\left(\frac{\text{mg substance}}{\text{kg food}}\right) = Dcoef * \frac{\text{Concentration}\left(\frac{\text{mg substance}}{\text{kg plastic}}\right) * \rho\left(\frac{\text{g plastic}}{cm^3}\right) * S(cm^2) * e(cm)}{M(\text{g food})}$$

$$\text{EDI}\left(\frac{\text{mg substance}}{\text{person} - \text{day}}\right) = m\left(\frac{\text{Kg ingested food}}{\text{person} - \text{day}}\right) * \text{Theoretical migration}\left(\frac{\text{mg substance}}{\text{kg food}}\right)$$

Where:

- $D_{coef}$: Diffusion coefficient. If 100% total migration is assumed, the diffusion coefficient is 1.
- $\rho\left(\frac{g}{cm^3}\right)$ : density of the polymer .
- $S(cm^2)$: packaging surface .

- e(cm): packaging thickness .
- M(g): product weight .
- m(kg): quantity of ingested food per person per day .

This series of parameters can be determined in the program parameters. The migration values are obtained assuming a total migration from the packaging to food. Moreover, the database contains information on each of the known substances. When a new test is recorded, the database extracts the following package of information for each of the identified substances:

- Legislation applicable to the substance (for example, in the form of SML: specific migration limit, mg substance/kg food, EU FCM 10/2011)
- Cramer classification (classification I, II, III carried out by the scientist Cramer, on the basis of a series of 33 questions related to the type of structure and atoms that make up the organic compound, TTC (Threshold Toxicological Concern, mg substance/person-day or mg substance/day-body weight, methodology accepted by competent bodies such as EFSA, to assess the safety of substances with an unknown toxicity that are present in food).
- CMR toxicity of the substance (checking whether the substance is listed as carcinogenic, mutagenic or reprotoxic on the list of compounds of very high concern SVHC).

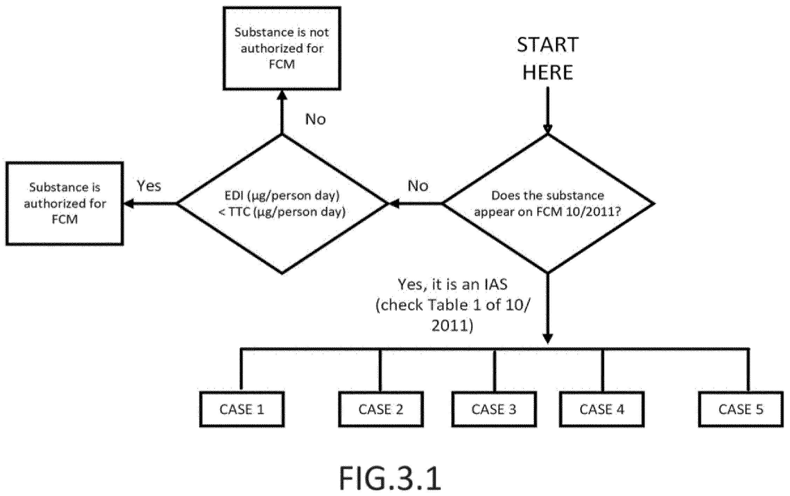

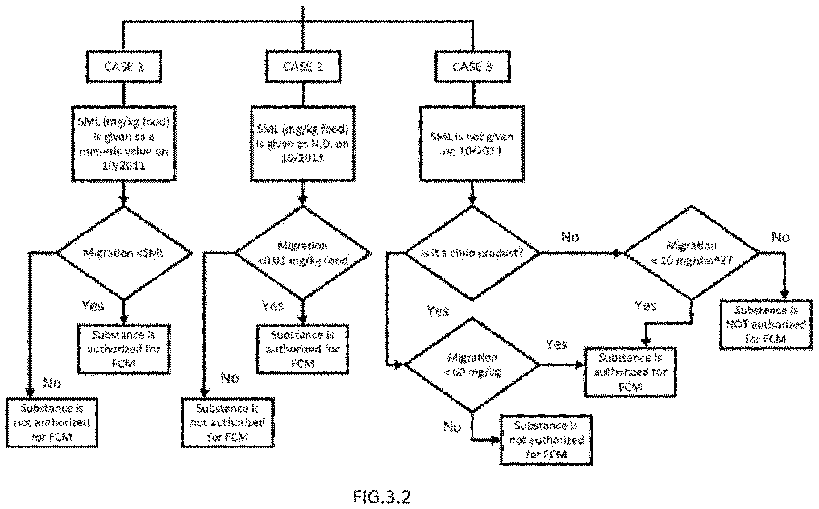

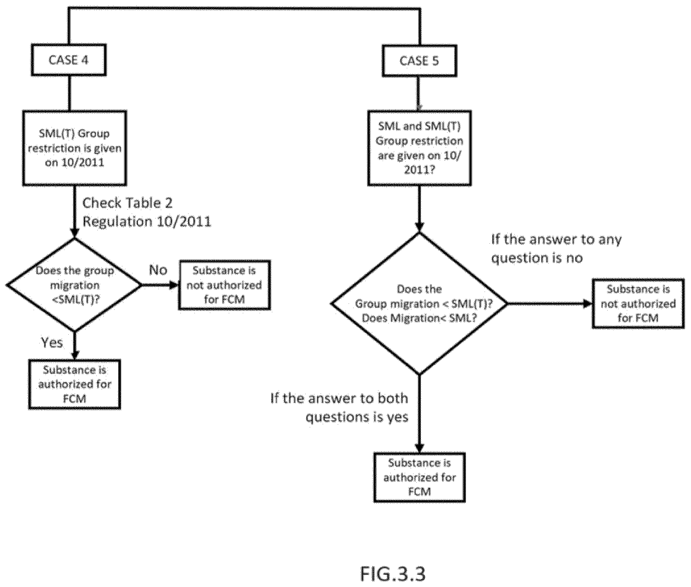

This data is used to verify whether the manufactured material would comply, for example, with the requirements established for material in contact with food. The criterion that is considered to carry out the risk test of a material is given in the decision tree that appears in figure 3, with its three images figure 3.1, figure 3.2 and figure 3.3.

Data can be fed by capturing an image of the plastic sample. The image taken by a microscope or multispectral camera, among others, will be subjected to processing by means of, for example, filters such as the fast local Laplacian filter, blur mask and non-local means.

Next, the different steps are listed:

- Collecting and preparing the data set of images of recycled plastics with different characteristics and recycled material content.
- Developing and programming technologies and functional modules.
- Training the image processing model.
- Assessing the performance of the image processing model based on simulated data.

Subsequently, the information obtained will be transmitted to the system configured with the model pre-trained with other images, for example, of a known recycling percentage. In this regard, the material is characterized in terms of concentration and in the case of mixtures of virgin/recycled material, it will predict the percentage of recycling in the sample. In this perspective, the system will correlate the recycled content with the chemical composition in order to adjust the extrusion recipe according to the needs of the final product.

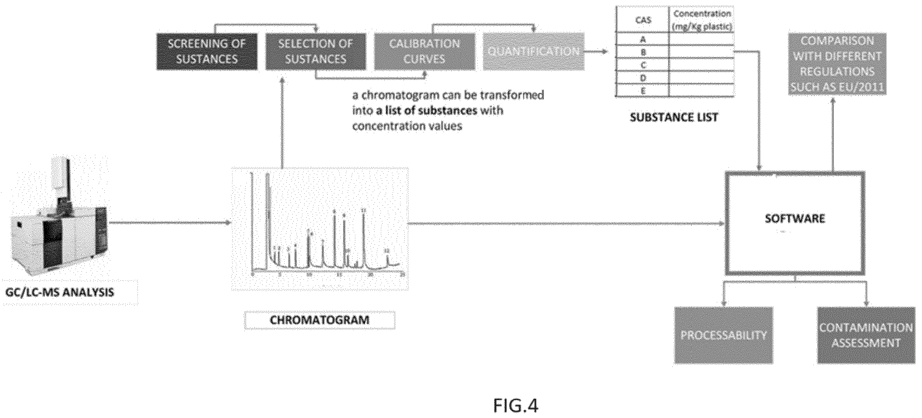

In the case of determining the chemical composition, the image obtained directly from the chromatogram can serve as a basis to feed the system, as can be seen in figure 4.

If the user has information on the true percentage of recycling in their sample, they will be able to enter this information into the program and retrain the model. Similarly, the efficiency of the algorithm will increase as the number of images used for model training increases. The algorithms used in training the model are based on Support Vector Machine (SVM) and Deep Learning.

Image processing is carried out in various formats, such as .jpg and .bmp, in addition to admitting other formats such as .png or .tiff. The image database will be hosted on the same computer on which the tool is used. The program can access images from both the computer and the camera in "live" mode. In the system, MATLAB's "image datastore" functionality will be used, facilitating the efficient management of large volumes of graphic data. As a result, an estimate of the percentage of recycling in the samples at hand will be generated.

Additionally, the effectiveness during the training step of the algorithm is assessed through the use of confusion matrices.

The possibility of redesigning is considered in the event that the initial results do not reach the desired degree of accuracy, and moreover, the application, among others, of neural networks directly on the set of data is not discarded.

Through virtual image storage called "Dataset", the images are stored in a library. At the same time, a .csv file is created with the names of the images in the Dataset, the path to the storage and the labels to be used for cross-validation.

Training was carried out using, among others, SVM (Support Vector Machine), Random Forest and Deep Learning algorithms. Image preprocessing is carried out both with LBP (Local Binary Pattern) and using pixels as characteristics, among others. The average results in terms of accuracy are listed below.

Table 1

| ALGORITHM | ACCURACY |
|---|---|
| SVM | 23.6 - 43% |
| SVM (preprocessing with LBP) | 17.0 - 34% |
| Random Forest | 45.2 - 62% |
| Random Forest (preprocessing with LBP) | 57.2 - 75% |
| Deep Learning | 30.0 - 52% |
| Deep Learning (preprocessing with LBP) | 59.0 - 87% |

The use of the aforementioned algorithms does not exclude the use of other alternative techniques, which could be selected based on the accuracy of the results and the characteristics of the available data.

By way of example, the training execution duration was less than one minute per fold for SVM and Random Forest with 140 images. With these same images, the range was several minutes per fold for Deep Learning.

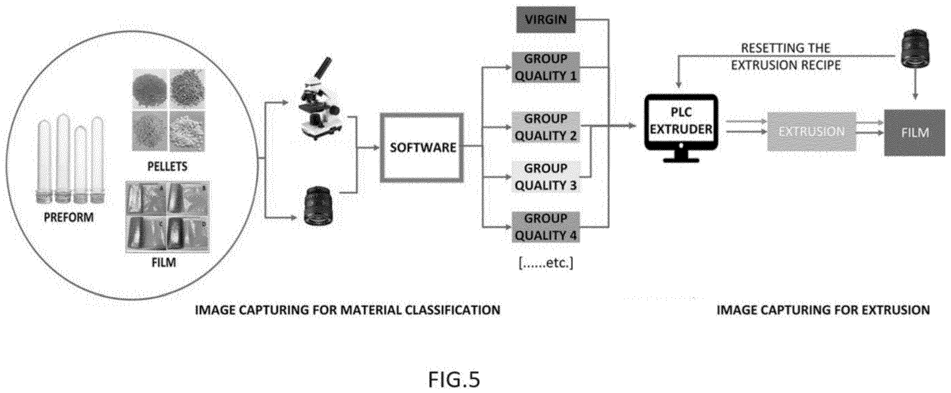

Figure 5 describes the method for classifying recycled material by capturing images, which have been previously correlated with various analytical techniques, including thermogravimetric and spectroscopic methods, among other techniques. These images are captured in various formats, such as film, pellets or preform.

During the manufacturing phase of the plastic film (extrusion process, using recycled material with a known level of contamination (LC), a recipe adjustment process is carried out, through communication between the image capturing device and the Programmable Logic Controller (PLC) of the extruder. The PLC is equipped with specialized software that allows readjusting the extruder based on the captured images and the level of contamination of the recycled material.

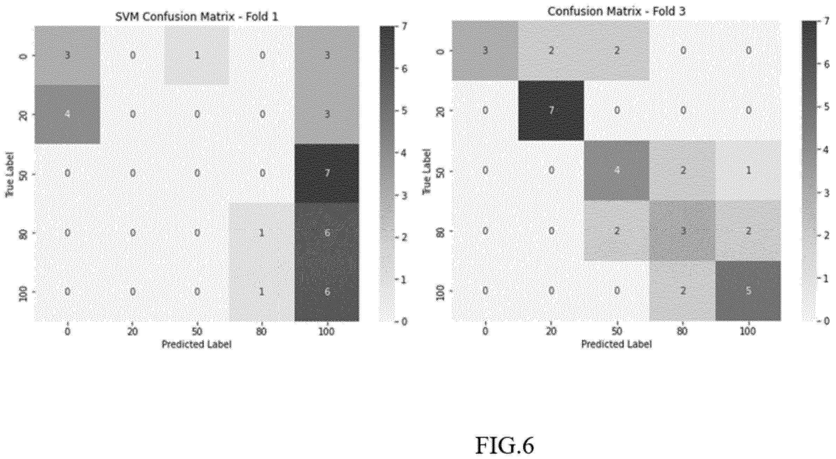

In order to evaluate the accuracy of the algorithms, confusion matrices are used, among others. These matrices mark the numbers of successes and failures in a table so that the predictions for each true label can be displayed, as can be seen in figure 6.

By way of example, figure 6 represents the confusion matrices obtained through SVM approximation (figure 6, left) and Random Forest (figure 6, right). It is clearly shown that the performance of Random Forest is superior to that of SVM. The Random Forest algorithm achieves higher accuracy for each label and, in the event of an error, the percentage of incorrect recycling (%PCr) is closer to reality. By contrast, SVM fails to generalize and is unable to identify the trend.

*Extrusion and batching based on the quality of the recycled material*

The batching system can be used for all types of extruders for the production of PET, LLDPE, LDPE, HDPE, PP, PLA, and EVA, among others. It involves preparing feed mixtures for the screw (or screws) in accordance with the algorithm described above. The input mixtures are composed of plastic pellets (LDPE, PET or others) and/or various additives. The plastics, in turn, can be of virgin or recycled origin. The quality of recycled plastic is the one with the most variability and that which dictates the need in this control system.

The great variability of the recycled raw material with different levels of contamination will have to be part of the output product with a homogeneous and unique level of contamination (LC) (imposed by customer). This desired LC is a reference value for controlling the extrusion process. The true LC value is the value calculated from the samples of raw material available for feeding the extruder. The objective of the system is to prepare the mixture in real time by adjusting the true LC value to the reference LC value.

It is important to note that the foregoing flowchart and block diagram referenced herein illustrate the architecture, functionality and operation of possible system implementations, methods and computing devices according to various embodiments of the present invention. In this sense, each block of the flowchart or block diagram can represent a module, segment or portion of instructions, which includes one or more executable instructions to implement the specified logical function(s). In some alternative implementations, the functions indicated in the block may occur out of the order indicated in the figures. For example, two blocks shown in succession can, in fact, substantially run concurrently, or the blocks can sometimes be executed in reverse order, depending on the functionality involved. It should also be noted that each block of the block diagrams and/or flowchart illustration, and the combinations thereof, may be implemented by systems based on special-purpose hardware that perform specified functions or acts or carry out combinations of special-purpose hardware and computer instructions.

More specifically, the present invention may be implemented as a programmatically executable process. Furthermore, the present invention may be implemented within a computing device in which programmatic instructions are stored and from which the programmatic instructions can be loaded into the memory of a data processing system and executed from it in order to carry out the previous programmatically executable process. Likewise, the present invention may be implemented within a data processing system adapted to load the programmatic instructions from a computing device and then execute the programmatic instructions in order to carry out the previous programmatically executable process.

To that end, the computing device is a non-transitory computer-readable storage means that retains or stores computer-readable program instructions. These instructions, when executed from the memory by one or more processing units of a data processing system, cause the processing units to perform different programmatic processes that exemplify different aspects of the programmatically executable process. In this sense, the processing units each include an instruction execution device, such as a central processing unit or "CPU" of a computer. One or more computers may be included in the data processing system. It should be noted that, although the CPU may be a single-core CPU, it should be understood that multiple-core CPUs can operate within the CPU and, in both cases, the instructions are loaded directly from the memory into the one or more cores of the one or more CPUs for their execution.

Apart from directly loading the instructions from the memory for their execution by one or more cores of a CPU or multiple CPUs, the computer-readable program instructions described herein can be retrieved, alternatively, from a computer communications network to the memory of a computer of the data processing system for their execution therein. Furthermore, only a portion of the program instructions can be retrieved in the memory via the computer communications network, while other portions can be loaded from the computer's persistent storage. Likewise, only a portion of the program instructions can be executed by one or more processing cores of one or more CPUs of one of the computers of the data processing system, while other portions may be executed cooperatively within a different computer of the data processing system that is co-located with the computer or located remotely from the computer via the computer communications network, sharing the computing results of both computers.

The structures, materials, acts and corresponding equivalents of all the step means or elements plus the function of the following claims are intended to include any structure, material or act to carry out the function in combination with other claimed elements as specifically claimed. The description of the present invention has been provided for illustrative and descriptive purposes, but it is not intended to be exhaustive or limited to the invention in the form disclosed. Many modifications and variations will be apparent to those skilled in the art without departing from the scope and spirit of the invention. The embodiment was chosen and described to better explain the principles of the invention and the practical application, and to enable others skilled in the art to understand the invention for various embodiments with various modifications as suitable for the particular use contemplated.

Having thus described the invention of the present application in detail and in reference to its embodiments, it will be obvious that modifications and variations are possible without departing from the scope of the invention defined in the attached claims, as follows:

## Claims

1. A method for manufacturing a thermoplastic material containing recycled material with a determined level of contamination by means of extrusion, comprising the steps of receiving one or more batches of a homogenized recycled material comprising polyethylene, or derivatives of polyethylene or a combination thereof, and **characterized in that** it comprises the steps of:

   (a) characterizing recycled input thermoplastic material by batches;
   (b) calculating the level of contamination (LC) from the characterization data of the material or generated images; and
   (c) calculating the percentage of recycled material to be introduced into the batching system of a plastic material extruder for the manufacture of a recycled plastic film with a known LC; and
   (d) extruding a thermoplastic material film, wherein (d.1) the contribution of virgin thermoplastic material is minimized; and (d.2) the batching is adjusted in real time according to the established level of contamination.

2. The method according to claim 1, wherein the characterization of the material comprises at least the following analyses: (a) analytical, configured for identification of pollutants using liquid chromatography mass spectrometry (LC-MS) or gas chromatography mass spectrometry (GC-MS); (b) physical, configured for measurement of color, turbidity, gel count or image analysis of the thermoplastic material; (c) mechanical, configured for measurement of tension, friction, o both; and (d) toxicity studies, configured for risk assessment for food contact applications.

3. The method according to claim 2, wherein in the analytical analysis, the homogenized thermoplastic material is subjected to an extraction process using an organic or aqueous solvent to carry out the study on identifying volatile, semi-volatile and non-volatile substances through the use of individual or joint techniques, GC-MS, LC-MS, ICP-MS.

4. The method according to claim 2, wherein in the toxicity analysis, the risk assessment is carried out according to whether the substance is carcinogenic, mutagenic or reprotoxic, if it appears as a substance of very high concern, as well as the maximum legal concentration level allowed.

5. The method according to claim 2, wherein the physical analysis comprises the characterization of the physical properties of the material through, at least, the measurement of color, opacity and gel count.

6. The method according to claim 2, wherein the mechanical analysis includes, at least, a friction and traction test of the recycled plastic material film.

7. The method according to any one of the preceding claims, wherein the calculation of the level of contamination from the characterization data of the material comprises the statistical analysis of the characterization data of the material by means of: (a) a supervised learning model using at least one selected from: (a.1) a neural network, (a.2) a supervised Bayesian model; or (a.3) a decision tree model; or (b) an unsupervised model with a target function that represents the desired concentration level in the final product and one or more constraints that represent the limits of the available concentrations.

8. The method according to claim 1, wherein in step (b) the level of contamination (LC) is calculated based on images generated using tools such as microscopes or multispectral cameras.

9. The method according to claim 1, wherein in step (b) the images are subjected to processing to normalize the data, wherein the initial image consists of the very chromatogram obtained during the analytical contamination test, establishing a correlation between the image and its level of contamination, there being preprocessing of the images using filters such as the fast local Laplacian filter, blur mask and non-local means.

10. The method according to claim 9, wherein the information obtained is subsequently transmitted to a processor with the model pre-trained with other images of a known recycling percentage, wherein the material is **characterized in** terms of concentration and wherein the recycled content is correlated with the chemical composition in order to adjust the extrusion recipe according to the needs of the final product.

11. The method according to claim 10, wherein the training of the algorithm is assessed through the use of confusion matrices.

```
┌─────────────────────────┐          ┌─────────────────────────┐
│ Preparation of calibrations │        │   Reception of plastic   │
│      of pollutants      │          │        samples          │
└─────────────────────────┘          └─────────────────────────┘
            │                                    │
            ▼                                    ▼
┌─────────────────────────┐          ┌─────────────────────────┐
│    Injection into GC-MS  │          │  Extraction of the pollutants │
└─────────────────────────┘          └─────────────────────────┘
            │                                    │
            ▼                                    ▼
┌─────────────────────────┐          ┌─────────────────────────┐
│    Elaboration of the   │          │  Preparation of the injection │
│    calibration lines    │          │          vials          │
└─────────────────────────┘          └─────────────────────────┘
            │                                    │
            │                                    ▼
            │                         ┌─────────────────────────┐
            │            ◄────────────│    Injection into GC-MS  │
            │                         └─────────────────────────┘
            ▼
```

| Quantitative analysis of samples (depending on extraction solvent) with specialized software | → | Export of result tables to spreadsheets | → | Calculation of the concentration of the samples (depending on the mass of plastic ) | → | Summary spreadsheet preparation for algorithm use |
|---|---|---|---|---|---|---|

FIG.1

FIG.2

FIG.3.1

FIG.3.2

FIG.3.3

FIG.4

FIG.5

FIG.6

## INFORME DE BÚSQUEDA INTERNACIONAL

| Solicitud internacional Nº |
|---|
| **PCT/ES2024/070103** |

### A. CLASIFICACIÓN DEL OBJETO DE LA SOLICITUD

*B29C 48/00*(2019.01)i; *B29C 48/92*(2019.01)i; *G01N 33/44*(2006.01)i; *B29B 17/00*(2006.01)i; *B29C 48/27*(2019.01)i; *B29C 48/275*(2019.01)i; *B29B 7/38*(2006.01)i; *B29B 7/72*(2006.01)i; *B29K 23/00*(2006.01)n; *B29K 67/00*(2006.01)n; *B29K 7/00*(2006.01)n; *B29K 105/00*(2006.01)n; *B29K 105/26*(2006.01)n; *B29L 7/00*(2006.01)n; *B29C 48/08*(2019.01)n

De acuerdo con la Clasificación Internacional de Patentes (CIP) o según la clasificación nacional y CIP.

### B. SECTORES COMPRENDIDOS POR LA BÚSQUEDA

Documentación mínima buscada (sistema de clasificación seguido de los símbolos de clasificación)

B29C; B29B; G01N; B29K; B29L

Otra documentación consultada, además de la documentación mínima, en la medida en que tales documentos formen parte de los sectores comprendidos por la búsqueda

Bases de datos electrónicas consultadas durante la búsqueda internacional (nombre de la base de datos y, si es posible, términos de búsqueda utilizados)

EPO-Internal

### C. DOCUMENTOS CONSIDERADOS RELEVANTES

| Categoría* | Documentos citados, con indicación, si procede, de las partes relevantes | Relevante para las reivindicaciones nº |
|---|---|---|
| X | US 2022227977 A1 (KLECZEK MONIKA [CA] ET AL) 21 julio 2022 (2022-07-21) | 1, 2, 5, 6, 8 |
| Y | * figuras 1-2; reivindicaciones 1, 9-15 y 19-23; párrafos 26-28 y 32; ejemplos * | 3, 4, 7, 9-11 |
| X | US 2005203656 A1 (ERNST TODD C [US] ET AL) 15 septiembre 2005 (2005-09-15) | 1-11 |
| Y | * figuras 1-17 y los párrafos correspondientes que figuran en la descripción; reivindicaciones 1-33; párrafos 3-13 * | 1-11 |
| X | US 2017298219 A1 (VORST KEITH [US] ET AL) 19 octubre 2017 (2017-10-19) | 1-11 |
| Y | * figuras 1-17; reivindicaciones 1-34; columna 2, líneas 7-59 * | 1-11 |
| X | WO 2014186292 A1 (DANES JEFFREY E [US]; VORST KEITH L [US]) 20 noviembre 2014 (2014-11-20) | 1-11 |
| Y | * figuras 1-14 y los pasajes correspondientes que figuran en la descripción; reivindicaciones 1-20; página 1, líneas 5-16; página 7, línea 19 hasta página 8, línea 24; página 8, línea 25 hasta página 9, línea 14; página 9, líneas 20-30; página 17, líneas 19-33; página 19, líneas 15-27, página 24, líneas 6-22 * | 1-11 |

☑ En la continuación del Recuadro C se relacionan otros documentos          ☑ Los documentos de familias de patentes se indican en el Anexo

| * | Categorías especiales de documentos citados: | "T" | documento ulterior publicado con posterioridad a la fecha de presentación internacional o de prioridad que no pertenece al estado de la técnica pertinente pero que se cita por permitir la comprensión del principio o teoría que constituye la base de la invención. |
|---|---|---|---|
| "A" | documento que define el estado general de la técnica no considerado como particularmente relevante. | | |
| "E" | solicitud de patente o patente anterior pero publicada en la fecha de presentación internacional o en fecha posterior. | "X" | documento particularmente relevante: la invención reivindicada no puede considerarse nueva o que implique una actividad inventiva por referencia al documento aisladamente considerado. |
| "L" | documento que puede plantear dudas sobre una reivindicación de prioridad o que se cita para determinar la fecha de publicación de otra cita o por una razón especial (como la indicada). | | |
| "O" | documento que se refiere a una divulgación oral, a una utilización, a una exposición o a cualquier otro medio. | "Y" | documento particularmente relevante: la invención reivindicada no puede considerarse que implique una actividad inventiva cuando el documento se asocia a otro u otros documentos de la misma naturaleza, cuya combinación resulta evidente para un experto en la materia. |
| "P" | documento publicado antes de la fecha de presentación internacional pero con posterioridad a la fecha de prioridad reivindicada. | "&" | documento que forma parte de la misma familia de patentes. |

| Fecha en que se ha concluido efectivamente la búsqueda internacional | Fecha de expedición del informe de búsqueda internacional |
|---|---|
| **26 junio 2024** | **30 julio 2024** |

| Nombre y dirección postal de la Administración encargada de la Funcionario autorizado búsqueda internacional | Funcionario autorizado |
|---|---|
| **European Patent Office** **p.b. 5818, Patentlaan 2, 2280 HV Rijswijk** **Países Bajos (Reino de los)** Nº de teléfono: **(+31-70)340-2040** Nº de fax: **(+31-70)340-3016** | **Okunowski, Françoise** Nº de teléfono: |

Formulario PCT/ISA/210 (segunda hoja) (Enero 2015)

**INFORME DE BÚSQUEDA INTERNACIONAL**

| Solicitud internacional N° |
| --- |
| **PCT/ES2024/070103** |

**C.    DOCUMENTOS CONSIDERADOS RELEVANTES**

| Categoría* | Documentos citados, con indicación, si procede, de las partes relevantes | Relevante para las reivindicaciones n° |
| --- | --- | --- |
| Y | ANDRAJU NAGABABU ET AL. "Machine-Learning-Based Predictions of Polymer and Postconsumer Recycled Polymer Properties: A Comprehensive Review" *APPLIED MATERIALS & INTERFACES*, US, Vol. 14. No. 38, 14 septiembre 2022 (2022-09-14), páginas 42771-42790 DOI: 10.1021/acsami.2c08301 ISSN: 1944-8244, XP093087534 <br> * página 42774, capítulo 1.2; páginas 42776-42781, capítulo 2; Figura 7 en la página 42780; páginas 42781-42784; Figura 8 n página 42782 * | 1-11 |
| Y | LI HANKE ET AL. "Machine learning directed discrimination of virgin and recycled poly(ethylene terephthalate) based on non-targeted analysis of volatile organic compounds" *JOURNAL OF HAZARDOUS MATERIALS, ELSEVIER, AMSTERDAM, NL*, Vol. 436, 11 mayo 2022 (2022-05-11), [recuperado el 2022-05-11] DOI: 10.1016/J.JHAZMAT.2022.129116 ISSN: 0304-3894, XP087102978 <br> * página 3, capítulos 2.2 y 2.3; páginas 3-4, capítulo 2.4, página 9, capítulo 4: figura 1 en la página 5; figura 2 en la página 6 * | 1-11 |
| T | ELLAM M S. "EXTRUSION CONTROL IN FILM MAKING" *MEASUREMENT AND CONTROL, INSTITUTE OF MEASUREMENT AND CONTROL. LONDON. GB*, Vol. 28, No. 1, 01 febrero 1995, Revisado 28 febrero 1995. , páginas 5-09 ISSN: 0020-2940, XP000505453 <br> * página 5: "Feedstocks"; páginas 5-6: "Feeding systems"; página 8: "Reclaim blend control"; page 9: "Manufacturing system integration" * | |

Formulario PCT/ISA/210 (segunda hoja) (Enero 2015)

**INFORME DE BÚSQUEDA INTERNACIONAL**
Información relativa a miembros de familias de patentes

Solicitud internacional N°

**PCT/ES2024/070103**

| Documento de patente citado en al informe de búsqueda | | | Fecha de publicación (día/mes/año) | Miembro(s) de la familia de patentes | | | Fecha de publicación (día/mes/año) |
|---|---|---|---|---|---|---|---|
| US | 2022227977 | A1 | 21 julio 2022 | CA | 3135927 | A1 | 19 noviembre 2020 |
| | | | | EP | 3969505 | A1 | 23 marzo 2022 |
| | | | | US | 2022227977 | A1 | 21 julio 2022 |
| | | | | WO | 2020229932 | A1 | 19 noviembre 2020 |
| US | 2005203656 | A1 | 15 septiembre 2005 | US | 2005203656 | A1 | 15 septiembre 2005 |
| | | | | US | 2007085229 | A1 | 19 abril 2007 |
| US | 2017298219 | A1 | 19 octubre 2017 | US | 2017298219 | A1 | 19 octubre 2017 |
| | | | | US | 2019248566 | A1 | 15 agosto 2019 |
| WO | 2014186292 | A1 | 20 noviembre 2014 | US | 2014332994 | A1 | 13 noviembre 2014 |
| | | | | WO | 2014186292 | A1 | 20 noviembre 2014 |

Formulario PCT/ISA/210 (anexo_familia de patentes) (Enero 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1055500 A